(19) **Europäisches Patentamt / European Patent Office / Office européen des brevets**

(11) **EP 4 602 961 A1**

(12) **EUROPEAN PATENT APPLICATION**
published in accordance with Art. 153(4) EPC

(43) Date of publication:
**20.08.2025 Bulletin 2025/34**

(21) Application number: **22964492.7**

(22) Date of filing: **04.11.2022**

(51) International Patent Classification (IPC):
**A24F 40/57** (2020.01)

(52) Cooperative Patent Classification (CPC):
**A24F 40/57**

(86) International application number:
**PCT/JP2022/041233**

(87) International publication number:
**WO 2024/095476 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Japan Tobacco Inc.**
**Tokyo 105-6927 (JP)**

(72) Inventor: **YOSHIDA, Ryo**
**Tokyo 130-8603 (JP)**

(74) Representative: **Hoffmann Eitle**
**Patent- und Rechtsanwälte PartmbB**
**Arabellastraße 30**
**81925 München (DE)**

(54) **AEROSOL GENERATION SYSTEM, CONTROL METHOD, AND PROGRAM**

(57)     [Problem] To provide a system which enables more appropriate control of a temperature at which an aerosol source is heated.

[Solution] An aerosol generation system comprising: a heating unit for heating an aerosol source; and a control unit for controlling the operation of the heating unit based on a parameter corresponding to the temperature of the heating unit, wherein the control unit controls the operation of the heating unit by repeating, in the stated order, a first step for measuring the parameter of the heating unit, and a second step for applying a voltage to the heating unit in a manner determined based on the parameter measured in the first step, and variably controls the duration of the second step.

EP 4 602 961 A1

**Description**

[Technical Field]

The present disclosure relates to an aerosol generation system, control method, and program.

[Background Art]

Inhalation devices that generate substances to be inhaled by a user, such as e-cigarettes and nebulizers, are in widespread use. For example, an inhalation device employs an aerosol source for generating an aerosol, and a substrate including a flavor source or the like for imparting a flavor component to the generated aerosol, to generate an aerosol to which the flavor component has been imparted. The user can enjoy the flavor by inhaling the aerosol to which the flavor component has been imparted, generated by the inhalation device. The action by which the user inhales the aerosol is also referred to below as "puffing" or a "puffing action".

In order to improve the flavor (also referred hereinafter as smoking taste) that the user tastes when puffing, it is preferable that the temperature at which the aerosol source is heated is appropriately controlled. In this regard, the following PTL 1 relates to a technique involving repeatedly applying a constant voltage to a heater to detect a resistance value of the heater and applying a voltage to the heater according to the detected resistance value, thereby controlling the temperature of the heater.

[Citation List]

[Patent Literature]

[PTL 1] JP 2022-520322 A

[Summary of Invention]

[Problem to be Solved by the Invention]

However, the technique disclosed in PTL 1 has only recently been developed, and there is still room for improvement in various aspects.

The present disclosure therefore takes account of the problems above, and the objective of the present disclosure lies in providing a system which enables more appropriate control of the temperature at which an aerosol source is heated.

[Solution to Problem]

To solve the above problem, one aspect of the present invention provides an aerosol generation system comprising: a heating unit for heating an aerosol source; and a control unit for controlling the operation of the heating unit based on a parameter corresponding to the temperature of the heating unit, wherein the control unit controls the operation of the heating unit by repeating, in the stated order, a first step for measuring the parameter of the heating unit, and a second step for applying a voltage to the heating unit in a manner determined based on the parameter measured in the first step, and variably controls the duration of the second step.

The control unit may control the duration of the second step based on the parameter measured in the first step.

The control unit may control the operation of the heating unit based on control information defining a time series transition of a target value of the parameter, and may control the duration of the second step based on the control information.

The period during which the operation of the heating unit is controlled based on the control information comprises, in the stated order, a first period during which the temperature of the heating unit is increased from or maintained at an initial temperature, a second period following the first period during which the temperature of the heating unit decreases, and a third period following the second period, and the control may cause the duration of the second step to differ between the first period and the third period.

The control unit may make the duration of the second step in the third period shorter than the duration of the second step in the first period.

The control unit may, in the third period, increase the duration of the second step in accordance with the passage of time.

The control unit may, in the first period, shorten the duration of the second step after the parameter measured in the first step reaches the specific target value.

The control unit may, in the first period, shorten the duration of the second step in accordance with the passage of time.

The control unit may control the duration of the second step based on the difference between the parameter measured in the first step and the target value defined in the control information.

The control unit may control the duration of the second step based on a manner of applying voltage to the heating unit in the second step.

The control unit may control the duration of the second step based on a user operation.

The aerosol generation system may further comprise a notification unit for notifying of information in accordance with the duration of the second step.

The aerosol generation system may further comprise a substrate containing the aerosol source.

Furthermore, to solve the above problem, another aspect of the present invention provides a control method executed by a computer for controlling an aerosol generation system, the aerosol generation system having a heating unit for heating an aerosol source, the control method including controlling the operation of the heating unit based on a parameter corresponding to the temperature of the heating unit, wherein controlling the operation of the heating unit includes controlling the operation of the heating unit, by repeating, in the stated order, a first step for measuring the parameter of the heating unit, and a second step for applying a voltage to the heating unit in a manner determined based on the parameter measured in the first step, and variably controlling the duration of the second step.

Furthermore, to solve the above problem, another aspect of the present invention provides a program executed by a computer for controlling an aerosol generation system, the aerosol generation system having a heating unit for heating an aerosol source, the program causing the computer to function as a control unit for controlling the operation of the heating unit based on a parameter corresponding to the temperature of the heating unit, wherein the control unit controls the operation of the heating unit, by repeating, in the stated order, a first step for measuring the parameter of the heating unit, and a second step for applying a voltage to the heating unit in a manner determined based on the parameter measured in the first step, and variably controls the duration of the second step.

[Advantageous Effects of Invention]

The present disclosure as described above provides a system which enables more appropriate control of the temperature at which an aerosol source is heated.

[Brief Description of Drawings]

Fig. 1 is a schematic diagram illustrating schematically a configuration example of an inhalation device.
Fig. 2 is a graph showing an example of a transition in temperature of a heating unit when temperature control is performed based on the heating profile shown in Table 1.
Fig. 3 is a graph for illustrating operational control of the heating units according to the present embodiment.
Fig. 4 is a flow chart showing an example of a processing flow executed in the inhalation device according to the present embodiment.

[Description of Embodiments]

Preferred embodiments of the present disclosure will now be described in detail with reference to the accompanying drawings. It should be noted that in the specification and the drawings, duplicate descriptions are omitted by using the same reference signs to denote constituent elements having substantially the same functional configuration.

<1. Example configuration of inhalation device>

An inhalation device is a device for generating a substance to be inhaled by a user. Hereinafter, the substance generated by the inhalation device will be described as being an aerosol. Alternatively, the substance generated by the inhalation device may be a gas.

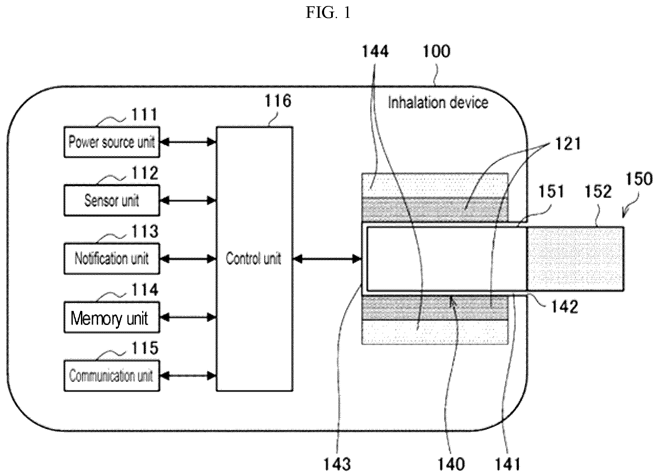

Fig. 1 is a schematic diagram illustrating schematically a configuration example of an inhalation device. As illustrated in fig. 1, an inhalation device 100 according to the present configuration example comprises a power source unit 111, a sensor unit 112, a notification unit 113, a memory unit 114, a communication unit 115, a control unit 116, heating units 121, an accommodating portion 140, and a heat insulating portion 144.

The power source unit 111 stores electric power. The power source unit 111 then supplies the electric power to each component of the inhalation device 100 in accordance with control performed by the control unit 116. The power source unit 111 may be configured, for example, by a rechargeable battery such as a lithium ion secondary battery.

The sensor unit 112 acquires various types of information relating to the inhalation device 100. As an example, the sensor unit 112 is configured by a pressure sensor such as a condenser microphone, a flow rate sensor or a temperature sensor, etc., and acquires values associated with inhalation by a user. As another example, the sensor unit 112 is configured by an input device, such as a button or switch, for accepting input of information from the user.

The notification unit 113 notifies the user of the information. The notification unit 113 is configured by a light emitting device that emits light, a display device that displays images, a sound output device that outputs sound, or a vibrating device that vibrates, for example.

The memory unit 114 stores various types of information for the operation of the inhalation device 100. The memory unit 114 is configured by a non-volatile storage medium such as a flash memory, for example.

The communication unit 115 is a communication interface capable of performing communication conforming to any wired or wireless communication standard. Examples of communication standards that may be used include standards that employ Wi-Fi (registered trademark), Bluetooth (registered trademark), BLE (Bluetooth Low Energy) (registered trademark), NFC (Near-Field Communication), or LPWA (Low Power Wide Area), for example.

The control unit 116 functions as an arithmetic processing device and a control device, and controls overall operation within the inhalation device 100 in accordance with various programs. The control unit 116 is realized by a CPU (Central Processing Unit) or an electronic circuit such as a microprocessor, for example.

The accommodating portion 140 has an internal space 141, and holds a stick-type substrate 150 while accommodating a portion of the stick-type substrate 150 in the internal space 141. The accommodating portion 140 has an opening 142 allowing the internal space 141 to communicate with the outside, and accommodates the stick-type substrate 150 that has been inserted into the internal space 141 from the opening 142. For example, the accommodating portion 140 is a cylindrical body comprising the opening 142 and a bottom portion 143 serving as a bottom surface, and defines a columnar internal space 141. An air flow path for supplying air to the internal space 141 is connected to the accommodating portion 140. An air inflow hole, which is an inlet for air into the air flow path, is disposed in a side surface of the inhalation device 100, for example. An air outflow hole serving as an outlet for air from the air flow path to the internal space 141 is disposed in the bottom portion 143, for example.

The stick-type substrate 150 comprises a substrate portion 151 and a mouthpiece portion 152. The substrate portion 151 includes an aerosol source. The aerosol source includes a tobacco-derived or non-tobacco-derived flavor component. If the inhalation device 100 is a medical inhaler such as a nebulizer, the aerosol source may include a drug. The aerosol source may, for example, be a liquid such as water or a polyhydric alcohol, for example glycerol or propylene glycol, containing the tobacco-derived or non-tobacco-derived flavor component, or may be a solid including the tobacco-derived or non-tobacco-derived flavor component. In a state in which the stick-type substrate 150 is being held in the accommodating portion 140, at least a portion of the substrate portion 151 is accommodated in the internal space 141, and at least a portion of the mouthpiece portion 152 protrudes from the opening 142. Then, when the user holds the mouthpiece portion 152 protruding from the opening 142 in their mouth and inhales, air flows into the internal space 141 via the air flow path, which is not illustrated in the drawings, and reaches the inside of the user's mouth together with the aerosol generated from the substrate portion 151.

The heating units 121 heat the aerosol source to atomize the aerosol source, thereby generating the aerosol. In the example illustrated in fig.1, the heating units 121 are configured in a film shape and are disposed so as to cover the outer periphery of the accommodating portion 140. Then, when the heating units 121 generate heat, the substrate portion 151 of the stick-type substrate 150 is heated from the outer periphery, generating the aerosol. The heating units 121 generate heat when supplied with electricity from the power source unit 111. By way of example, electricity may be supplied when the sensor unit 112 detects that the user has started inhaling and/or that predetermined information has been input. The supply of electricity may then be stopped when the sensor unit 112 detects that the user has finished inhaling and/or that predetermined information has been input.

The heat insulating portion 144 prevents heat transfer from the heating units 121 to other components. For example, the heat insulating portion 144 is configured from a vacuum heat insulating material or an aerogel heat insulating material, or the like.

A configuration example of the inhalation device 100 has been described above. The inhalation device 100 is, of course, not limited to the configuration described above, and may adopt various configurations, such as those illustrated below by way of example.

As one example, the heating unit 121 may have a blade-like form and may be arranged so as to protrude into the internal space 141 from the bottom portion 143 of the accommodating portion 140. In that case, the blade-like heating unit 121 is inserted into the substrate portion 151 of the stick-type substrate 150 and heats the substrate portion 151 of the stick-type substrate 150 from the inside. As another example, the heating unit 121 may be arranged so as to cover the bottom portion 143 of the accommodating portion 140. Furthermore, the heating unit 121 may be configured by a combination of two or more from among a first heating unit covering the outer circumference of the accommodating portion 140, a blade-like second heating unit, and a third heating unit covering the bottom portion 143 of the accommodating portion 140.

As another example, the accommodating portion 140 may comprise an opening/closing mechanism such as a hinge for opening/closing part of a casing that forms the internal space 141. By opening/closing the casing, the accommodating portion 140 may then receive and grip the stick-type substrate 150 which has been inserted into the internal space 141. In that case, the heating units 121 may be provided on the part of the accommodating portion 140 gripping the stick-type substrate 150, and may heat the stick-type substrate 150 while pressing same.

The combination of the inhalation device 100 and the stick-type substrate 150 generates an aerosol to be inhaled by the user. The combination of the inhalation device 100 and the stick-type substrate 150 may be considered to be an aerosol generation system.

<2. Technical features>

(1) Heating profile

The control unit 116 controls the operation of the heating units 121 based on the heating profile. Control of the operation of the heating units 121 is achieved by controlling the supply of power from the power source unit 111 to the heating units 121. The heating units 121 heat the stick-type substrate 150 using power supplied from the power source unit 111.

The heating profile is control information for controlling the temperature at which the aerosol source is heated. The heating profile may be control information for controlling the temperature of the heating units 121. By way of example, the heating profile may comprise a target value of the temperature at which the aerosol source is heated (also referred hereinafter as the target temperature). The target temperature may vary depending on the elapsed time since the start of heating, in which case the heating profile comprises information defining a time series transition of the target temperature. As another example, the heating profile may comprise a parameter (hereinafter also referred to as a power supply parameter) defining how power is supplied to the heating units 121. The power supply parameters include, for example, a voltage applied to the heating units 121, ON/OFF of the power supply to the heating units 121, or a method of feedback control to be employed. ON/OFF of the power supply to the heating units 121 may be considered as ON/OFF of the heating units 121.

The control unit 116 controls the operation of the heating units 121 such that the temperature of the heating units 121 transitions similarly to the target temperature defined in the heating profile. The heating profile is typically designed such that, when the user inhales the aerosol generated from the stick-type substrate 150, the flavour tasted by the user is optimized. Thus, by controlling the operation of the heating units 121 based on the heating profile, the flavor tasted by the user can be optimized.

The temperature control of the heating units 121 can be achieved by known feedback control, for example. The feedback control may be a Proportional-Integral-Differential Controller (PID) control, for example. The control unit 116 may cause power from the power supply unit 111 to be supplied to the heating units 121 in the form of pulses by pulse width modulation (PWM) or pulse frequency modulation (PFM). In that case, the control unit 116 can perform temperature control of the heating units 121 by adjusting the pulse width or frequency of the power pulse to control the duty ratio in feedback control. Alternatively, the control unit 116 may perform simple ON/OFF control in feedback control. For example, the control unit 116 may perform heating by the heating units 121 until the temperature of the heating units 121 reaches the target temperature, interrupt heating by the heating units 121 when the temperature of the heating units 121 reaches the target temperature, and resume heating by the heating units 121 when the temperature of the heating units 121 falls below the target temperature.

As an example, the temperature of the heating units 121 can be determined by measuring or estimating the electrical resistance of the heating units 121 (more precisely the resistive heating elements constituting the heating units 121). This is because the electrical resistance value of the resistive heating element varies with temperature. The electrical resistance of the resistive heating element can be estimated by measuring the amount of voltage drop at the resistive heating element, for example. The amount of voltage drop at the resistive heating element can be measured by a voltage sensor measuring a potential difference applied to the resistive heating element.

The period from the start of the process of generating an aerosol using the stick-type substrate 150 to the end is also referred to hereinafter as a heating session. In other words, a heating session is a period of time during which the operation of the heating units 121 is controlled based on the heating profile. The beginning of the heating session is the timing at which heating based on the heating profile is started. The end of the heating session is the timing at which a sufficient amount of aerosol is no longer generated. The heating session comprises a pre-heating period and a puffable period following the pre-heating period. The puffable period is the period of time during which a sufficient amount of aerosol is expected to be generated. The pre-heating period is the period from when heating is started until the puffable period is started. Heating performed in the pre-heating period is also referred to as pre-heating.

An example of the heating profile is shown in Table 1 below.

[Table 1]

| Table 1. One example of heating profile | | | | |
|---|---|---|---|---|
| Period | | | Time series transition of target temperature | Time series transition of power supply parameter |
| Name | Division | Duration | | |
| Initial heating period | STEP 0 | - | Increase up to 295 °C (no time control) | ON |
| | STEP 1 | 20 Seconds | Maintain 295°C | ON |
| | STEP 2 | 20 Seconds | Maintain 205°C | ON |
| Intermediate temperature -drop period | STEP 3 | - | Decrease down to 230°C (no time control) | OFF |
| Re-heating period | STEP 4 | 40 Seconds | Maintain 230°C | ON |
| | STEP 5 | 40 Seconds | Increase up to 260°C | ON |
| | STEP 6 | 40 Seconds | Maintain 260°C | ON |
| Heating end period | STEP 7 | 20 Seconds | - | OFF |

As shown in Table 1, the heating profile may be divided into a plurality of periods, and a time series transition of the target temperature and a time series transition of the power supply parameters are defined in each period. In the example shown in Table 1, the heating profile is divided into a total of eight periods of step 0 to step 7. In each step, a time series transition of the target temperature and a time series transition of the power supply parameters are defined.

As shown in Table 1, the heating profile includes information for controlling the heating units 121 in each of an initial heating period, an intermediate temperature-drop period, a re-heating period, and a heating end period. The initial heating period is a period during which the temperature of the heating units 121 is increased from or maintained at the predetermined temperature, and is an example of a first period. The initial heating period consists of step 0 to step 2. The intermediate temperature-drop period is a period following the initial heating period and during which the temperature of the heating units 121 drops, and is an example of a second period. The intermediate temperature-drop period consists of step 3. The re-heating period is a period following an intermediate temperature-drop period, during which the temperature of the heating units 121 is increased or maintained, and is an example of a third period. The re-heating period consists of step 4 to step 6. The heating end period follows the re-heating period and is the period during which the temperature of the heating units 121 decreases. The heating end period consists of step 7. By the heating session sequentially comprising an initial heating period, an intermediate temperature-drop period, and a re-heating period, it is possible to shorten the pre-heating period, prevent rapid consumption of the aerosol source, and optimize the smoking taste delivered to the user, as described later.

In each step, time control may be implemented. Time control is control that terminates a step with the elapsing of a predetermined period (i.e., the duration set for each step) as the trigger. Note that when time control is implemented, the rate of change of the temperature of the heating units 121 may be controlled so that the temperature of the heating units 121 reaches the target temperature at the end of the duration. Alternatively, it may be taken as a gradual change of the target temperature throughout the entirety of the step. Alternatively, if time control is implemented, the temperature of the heating units 121 may be controlled such that the temperature of the heating units 121 reaches the target temperature partway through the duration and thereafter the temperature of the heating units 121 maintains the target temperature until the duration elapses. In the example shown in Table 1 above, time control is performed in steps 1, 2 and 4 to 7.

In each step, time control may not be implemented. If no time control is implemented, the step ends when the temperature of the heating units 121 has reached the predetermined temperature (i.e., the target temperature set for each step) as the trigger. As such, the duration of the step in which no time control is implemented increases or decreases with the rate of temperature change. In the example shown in Table 1 above, no time control is performed in steps 0 and 3.

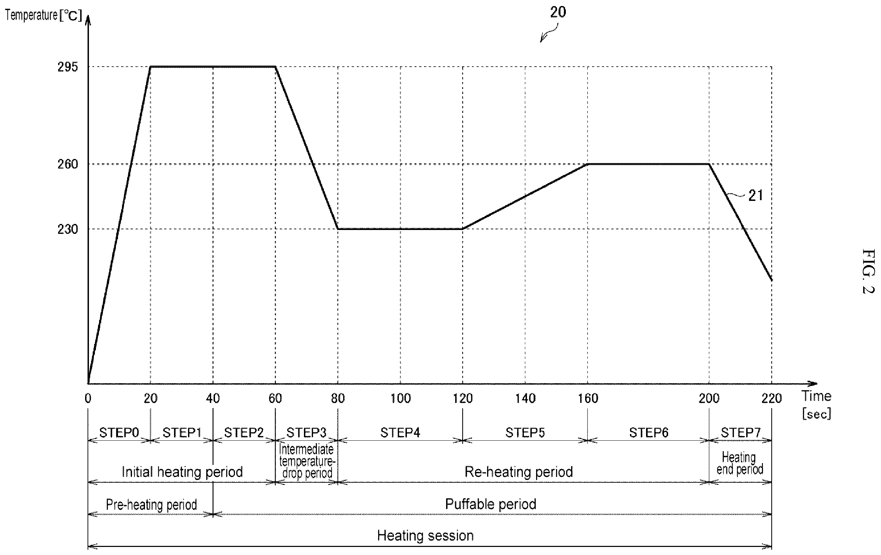

The transition of the temperature of the heating units 121 when the control unit 116 performs temperature control according to the heating profile shown in Table 1 will be described with reference to fig. 2. Fig. 2 is a graph showing an example of a transition in temperature of the heating units 121 when temperature control is performed based on the heating profile shown in Table 1. The horizontal axis of graph 20 is time in seconds. The vertical axis of graph 20 is the temperature of the heating units 121. Line 21 shows the transition of the temperature of the heating units 121. As shown in fig. 2, the temperature of the heating units 121 is transitioning in a similar manner to the transition of the target temperature defined in the heating profile. An example of a heating profile will be described below with reference to Table 1 and fig. 2.

As shown in Table 1 and fig. 2, in step 0, the temperature of the heating units 121 increases from the initial temperature to 295°C. The initial temperature is the temperature of the heating units 121 at the beginning of heating. In step 0, no time control is implemented. As such, step 0 ends with the temperature of the heating units 121 reaching 295°C as the trigger. In the example shown in fig. 2, step 0 ends in 20 seconds. Thereafter, in steps 1 and 2, the temperature of the heating units 121 is maintained at 295°C. The pre-heating period ends at the end of step 1, and the puffable period starts at the start of step 2.

A shorter pre-heating time is desirable for the user. However, if the stick-type substrate 150 is not sufficiently heated, moisture may not evaporate fully inside the stick-type substrate 150 and remain therein. If the user then takes a puff, hot vapor may be delivered to the user's mouth. As such, it is desirable to rapidly increase the temperature of the heating units 121 until it reaches 295°C in step 0 and to ensure a certain duration of steps 1 and 2.

As shown in Tables 1 and 2, in step 3, the temperature of the heating units 121 drops to 230°C. In step 3, no time control is implemented. As such, step 3 ends with the temperature of the heating units 121 reaching 230°C as the trigger. In the example shown in fig. 2, step 3 ends in 20 seconds. In step 2, the power supply to the heating units 121 is switched OFF. Therefore, the temperature of the heating units 121 can be reduced at the maximum speed. In this way, reducing the temperature of the heating units 121 partway through the heating session can prevent rapid consumption of the aerosol source. As a result, it is possible to prevent depletion of the aerosol source partway through the heating session.

As shown in Table 1 and fig. 2, next, from step 4 to step 6, the temperature of the heating units 121 increases gradually to 260°C. In this way, gradually increasing the temperature of the heating units 121 makes it possible to reduce power consumption during the entire heating session while maintaining an amount of aerosol generation.

As shown in Table 1 and fig. 2, in step 7, the temperature of the heating units 121 decreases. In step 7, the power supply to the heating units 121 is switched OFF. In step 7, the duration is defined, while the target temperature is not defined. Thus, step 7 ends with the end of the duration as the trigger. In step 7, a sufficient amount of aerosol may be generated due to the residual heat of the stick-type substrate 150. Thus, in this example, with the end of step 7, the puffable period, i.e., the heating session, ends.

The notification unit 113 may notify the user of information indicative of the timing at which the pre-heating ends. For example, the notification unit 113 notifies the user of information announcing the end of the pre-heating period before the pre-heating period ends, or notifies the user of information indicating that the pre-heating has ended at the timing at which the pre-heating has ended. The notification to the user can be by lighting an LED or vibrating, or the like, for example. By referring to such notification, the user is able to take a puff immediately after the end of the pre-heating.

Similarly, the notification unit 113 may notify the user of information indicative of when the puffable period ends. For example, the notification unit 113 notifies the user of information announcing the end of the puffable period before the puffable period ends, or notifies the user of information indicating that the puffable period has ended at the timing at which the puffable period has ended. The notification to the user can be by lighting an LED or vibrating, or the like, for example. By referring to such notification, the user is able to take a puff until the end of the puffable period.

Note that the heating profile described above is only an example and various other examples are conceivable. By way of example, the number of steps, the duration of each step, and the target temperature may be modified accordingly.

(2) Temperature measurement and PWM control

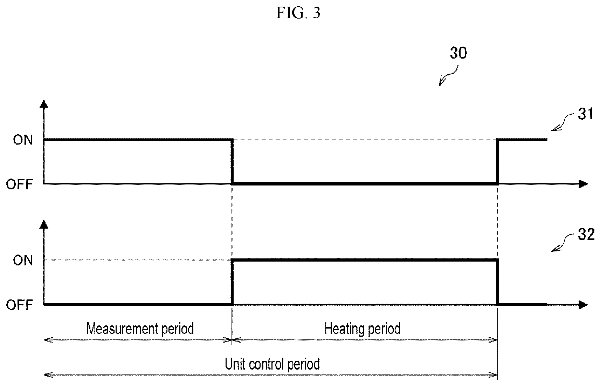

The control unit 116 controls the operation of the heating units 121 by repeating, in the stated order, a first step for measuring the temperature of the heating units 121 and a second step for applying a voltage to the heating units 121 in a manner determined based on the temperature of the heating units 121 measured in the first step. More specifically, in the first step, the control unit 116 applies a voltage to the heating units 121, measures an electrical resistance of the heating units 121, and measures the temperature of the heating units 121 based on the measured electrical resistance of the heating units 121. The control unit 116 then determines a duty ratio of the voltage applied to the heating units 121 in the second step as the manner of applying voltage to the heating units 121 in the second step based on the measured temperature of the heating units 121 and the target temperature defined in the heating profile. The control unit 116 then controls the power source unit 111 to apply a voltage of a pulse width or frequency corresponding to the determined duty ratio to the heating units 121 in a second step following the first step. The control unit 116 repeatedly executes a control block consisting of the first step and the second step. Such an arrangement allows the temperature of the heating units 121 to be transitioned as defined in the heating profile. In the following, the temperature of the heating units 121 shall be measured by the first step unless mentioned otherwise. The control block is described in detail with reference to fig. 3.

Fig. 3 is a graph for illustrating operational control of the heating units 121 according to the present embodiment. A graph 30 shows ON/OFF of the voltage applied to the heating units 121 at the control block. The unit control period is the period during which one control block is executed. The unit control period includes, in the stated order, a measurement period, which is the period during which the first step is performed, and a heating period, which is the period during which the second step is performed. The graph 30 includes a graph 31 and a graph 32. Graph 31 shows ON/OFF of the application of voltage for the first step. Graph 32 shows ON/OFF of the application of voltage for the second step. As shown in graph 31 and graph 32, after the voltage for the first step is applied in the measurement period, the voltage for the second step is applied in the heating period.

Note that the voltage applied to the heating units 121 in the measurement period may be weak compared to the voltage applied to the heating units 121 in the heating period. Furthermore, the duty ratio in the measurement period may be set to a low value, such as 1%. This can ensure that the temperature of the heating units 121 is not increased during the measurement period.

Conventionally, the lengths of the measurement period and the heating period were fixed. As such, there were times when it was difficult to appropriately control the temperature of the heating units 121. As an example, even if the duty ratio in the heating period was set to 100%, the duty ratio was reduced by an amount equivalent to the measurement period for the whole unit control period. As another example, the temperature of the heating units 121 was not measured during the heating period, and thus temperature tracking was reduced by an amount equivalent to the heating period. For example, if a puff was taken during the heating period, there would be a time lag equivalent to the heating period in length from the reduction in temperature of the heating units 121 with the puff until returning to the original state.

Thus, the control unit 116 according to the present embodiment controls the length of the heating period (i.e., the duration of the second step) in a variable manner. This configuration makes it possible to more appropriately control the temperature of the heating units 121.

The control unit 116 may control the length of the heating period based on the heating profile. In particular, the control unit 116 may control the length of the heating period depending on the progress based on the heating profile. According to such an arrangement, it is possible to optimize the length of the heating period depending on the progress of the control based on the heating profile.

In particular, the control unit 116 may cause the length of the heating period to differ between the initial heating period and the re-heating period. In the initial heating period, a rapid increase in the temperature of the heating units 121 is required to shorten the pre-heating period. On the other hand, in the re-heating period, high temperature tracking is required to recover more quickly from the drop in temperature of the heating units 121 associated with a puff. In this regard, according to such an arrangement, it is possible to optimize the length of the heating period both in the initial heating period and the re-heating period.

Specifically, the control unit 116 causes the length of the heating period in the re-heating period to be less than the length of the heating period in the initial heating period. As an example, the control unit 116 may set the length of the heating period to 196 ms in the initial heating period. The control unit 116 may then set the length of the heating period to 46 ms in the re-heating period.

In the following, the length of the measurement period shall be fixed at 4 ms. In addition, to simplify the calculation, the duty ratio in the measurement period is assumed to be 0%. If the length of the heating period is set to 46 ms, the duty ratio over the whole unit control period remains at 92% even if the duty ratio for the heating period is 100%. In this regard, if the length of the heating period in the initial heating period is set to 196 ms, the duty ratio over the whole unit control period can be increased up to a maximum of 98%. As a result, it is possible to rapidly increase the temperature of the heating units 121 in the initial heating period and shorten the pre-heating period. Furthermore, according to such a configuration, the interval between the measurement period and the next measurement period can be reduced from 196 ms in the initial heating period to 46 ms in the re-heating period. As a result, it is possible to improve the temperature tracking of the heating units 121 during the re-heating period.

(3) Process Flow

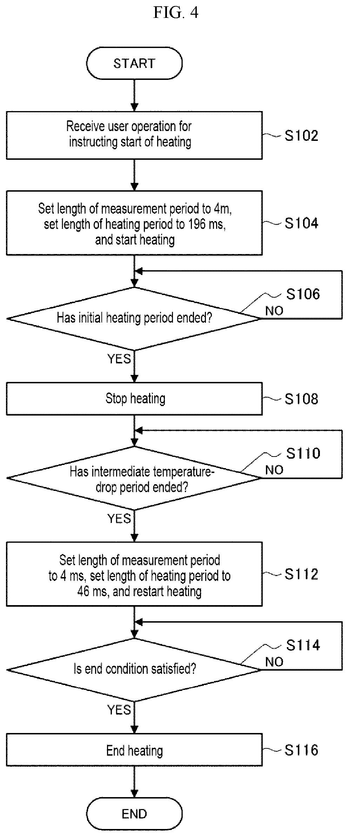

An example of the process flow performed in the inhalation device 100 according to the present embodiment will be described below with reference to fig. 4. Fig. 4 is a flow chart showing an example of a process flow executed in the inhalation device 100 according to the present embodiment.

As shown in fig. 4, initially, the sensor unit 112 receives a user operation for instructing the start of heating (step S102). An example of a user operation for instructing the start of heating is an operation on the inhalation device 100, such as operating a switch, etc. provided to the inhalation device 100. Another example of the user operation for instructing the start of heating is to insert a stick-type substrate 150 into the inhalation device 100.

Next, the control unit 116 sets the length of the measurement period to 4 ms and the length of the heating period to 196 ms to initiate heating based on the heating profile (step S104). For example, the control unit 116 controls the duty ratio of the voltage applied to the heating units 121 in the heating period based on the target temperature in the initial heating period and the temperature of the heating units 121 measured in the measurement period.

The control unit 116 then determines whether or not the initial heating period has ended (step S106). If it is determined that the initial heating period has not ended (step S106: NO), the control unit 116 continues heating by the heating units 121 until it is determined that the initial heating period has ended.

When it is determined that the initial heating period has ended (step S106: YES), the control unit 116 temporarily stops heating (step S108). For example, the control unit 116 stops the application of voltage to the heating units 121 across the whole unit control period.

The control unit 116 then determines whether or not the intermediate temperature-drop period has ended (step S110). For example, the control unit 116 determines that the intermediate temperature-drop period has ended when the temperature of the heating units 121 drops to the target temperature of the intermediate temperature-drop period. In the intermediate temperature-drop period, the temperature of the heating units 121 can be detected by a thermistor or the like arranged in the vicinity of the heating units 121, for example. If it is determined that the intermediate temperature-drop period has not ended (step S110: NO), the control unit 116 continues to monitor the temperature of the heating units 121 until it is determined that the intermediate temperature-drop period has ended.

If it is determined that the intermediate temperature-drop period has ended (step S110: YES), the control unit 116 sets the length of the measurement period to 4 ms, sets the length of the heating period to 46 ms, and resumes heating (step S112).

The control unit 116 then determines whether or not an end condition has been satisfied (step S114). An example of the end condition is that the heating session has ended. Another example of the end condition is that the predetermined number of puffs since the start of heating has been reached.

If the end condition is determined not to be satisfied (step S114: NO), the control unit 116 continues heating by the heating units 121 until it is determined that the end condition has been satisfied.

On the other hand, if the end condition is determined to be satisfied (step S114: YES), the control unit 116 ends heating based on the heating profile (step S116). After that, the process ends.

<3. Supplementary information>

Although preferred embodiments of the present disclosure have been described in detail above with reference to the accompanying drawings, the present disclosure is not limited to such examples. It is obvious that a person having an ordinary level of knowledge in the technical field to which the present disclosure belongs could conceive of various modified examples or variations within the scope of the technical concepts set forth in the claims, and these modified examples and variations will naturally be understood to fall within the technical scope of the present disclosure.

(1) First variant example

In the re-heating period, further control of the length of the heating period may be performed.

The higher the temperature of the heating units 121, the greater the divergence between the outside air entering the inhalation device 100 with a puff and the temperature of the heating units 121, and the greater the reduction in the temperature of the heating units 121 with the puff. Then, after the temperature of the heating units 121 decreases with the puff, the longer the temperature of the heating units 121 is diverged from the target temperature, the worse the smoking taste may be. As such, it is desirable that the drop in temperature of the heating units 121 associated with a puff is quickly recovered.

The control unit 116 may therefore control, in the re-heating period, the length of the heating period based on the temperature of the heating units 121. In detail, the control unit 116 may increase the length of the heating period as the temperature of the heating units 121 becomes higher, and may decrease the length of the heating period as the temperature of the heating units 121 becomes lower. According to such a configuration, in the re-heating period, it is possible to recover quickly from the drop in temperature of the heating units 121 associated with a puff.

It is conceivable that the higher the target temperature is, the higher the temperature of the heating units 121 is. As such, the control unit 116 may control, in the re-heating period, the length of the heating period based on the target temperature. In detail, the control unit 116 may increase the length of the heating period as the target temperature becomes higher, and may decrease the length of the heating period as the target temperature becomes lower. For example, in the example shown in fig. 2, the control unit 116 may set the length of the heating period in step 4 to 46 ms and the length of the heating period in step 5 to 56 ms, and may set the length of the heating period in step 6 to 66 ms. According to such a configuration, in the re-heating period, it is possible to recover quickly from the drop in temperature of the heating units 121 associated with a puff.

Alternatively, the control unit 116 may control, in the re-heating period, the length of the heating period based on the difference between the temperature of the heating units 121 and the target temperature. In detail, the control unit 116 may set the length of the heating period to be shorter as the difference between temperature of the heating units 121 and the target temperature becomes smaller, and may set the length of the heating period to be longer as the difference between the temperature of the heating units 121 and the target temperature becomes larger. For example, the control unit 116 may set the length of the heating period to 96 ms during the re-heating period, limited to when the temperature of the heating units 121 drops significantly from the target temperature following a puff, while normally setting the length of the heating period to 46 ms. According to such a configuration, in the re-heating period, it is possible to recover quickly from the drop in temperature of the heating units 121 associated with a puff.

Here, the duty ratio in the heating period is determined based on the difference between the temperature of the heating units 121 measured in the measurement period and the target temperature defined in the heating profile. As such, the control unit 116 may control, in the re-heating period, the length of the heating period based on the duty ratio in the heating period. In detail, the control unit 116 may set the length of the heating period to be shorter as the duty ratio becomes smaller, and may set the length of the heating period to be longer as the duty ratio becomes larger. Such a configuration also makes it possible to recover quickly from the drop in temperature of the heating units 121 associated with a puff in the re-heating period.

From another point of view, the more that time passes from the start of heating, the warmer the heating units 121 become to the core, and thus the temperature drop width of the heating units 121 associated with a puff can become large. Thus, the control unit 116 may increase the length of the heating period according to the passage of time in the re-heating period. For example, the control unit 116 may set the length of the heating period in the re-heating period according to the following equation (1). Note that T1 is the length of the heating period in the re-heating period, $\alpha$ is an optional coefficient, and t1 is the elapsed time since the start of the re-heating period. Such a configuration also makes it possible to recover quickly from the drop in temperature of the heating unit 121 associated with a puff in the re-heating period.

$$T1 = 46\,[\text{ms}] + \alpha \times t1\,[\text{ms}] \dots(1)$$

(2) Second variant example

In the initial heating period, further control of the length of the heating period may be performed.

In the initial heating period, the temperature of the heating units 121 rises rapidly, and thus there is a risk of a phenomenon in which the temperature of the heating units 121 exceeds the target temperature, or so-called overshoot. When overshoot occurs, the smoking taste may deteriorate. It is therefore desirable to prevent the occurrence of overshoot.

The control unit 116 may therefore control, in the initial heating period, the length of the heating period based on the temperature of the heating units 121. In detail, the control unit 116 may shorten the length of the heating period after the temperature of the heating units 121 reaches a specific target temperature in the initial heating period. For example, in the example shown in fig. 2, the control unit 116 may, in step 0, set the length of the heating period to 196 ms until the temperature of the heating units 121 reaches 280°C, which is in the vicinity of the target temperature of 295°C. The control unit 116 may then, in step 0, set the length of the heating period to 96 ms after the temperature of the heating units 121 has reached 280°C. Such a configuration makes it possible to prevent the occurrence of overshoot in the initial heating period.

Alternatively, the control unit 116 may control, in the initial heating period, the length of the heating period based on the difference between the temperature of the heating units 121 and the target temperature. In detail, the control unit 116 may set the length of the heating period to be shorter as the difference between temperature of the heating units 121 and the target temperature becomes smaller, and may set the length of the heating period to be longer as the difference between the temperature of the heating units 121 and the target temperature becomes larger. With such a configuration, the closer the temperature of the heating units 121 is to the target temperature of 295°C, the greater the temperature tracking, and thus it is possible to prevent the occurrence of overshoot in the initial heating period.

Here, the duty ratio in the heating period is determined based on the difference between the temperature of the heating units 121 measured in the measurement period and the target temperature defined in the heating profile. As such, the control unit 116 may control, in the initial heating period, the length of the heating period based on the duty ratio in the heating period. In detail, the control unit 116 may set the length of the heating period to be shorter as the duty ratio becomes smaller, and may set the length of the heating period to be longer as the duty ratio becomes larger. Such a configuration also makes it possible to prevent the occurrence of overshoot in the initial heating period.

As a simpler control method, the control unit 116 may shorten the length of the heating period according to the passage of time in the initial heating period. For example, the control unit 116 may set the length of the heating period in the initial heating period according to the following equation (2). Note that T2 is the length of the heating period in the initial heating period, $\beta$ is an optional coefficient, and t2 is the elapsed time since the start of the initial heating period. According to such a configuration, it is possible to prevent the occurrence of overshoot in the initial heating period.

$$T2 = 196[\text{ms}] - \beta \times t2[\text{ms}] \dots(2)$$

(3) Third variant example

The control unit 116 may control the length of the heating period based on the user operation. By way of example, the inhalation device 100 may operate in one of the following operating modes: a normal mode in which the length of the heating period is set based on the embodiment or the various variant examples mentioned above; and a boost mode in which the length of the heating period is extended from the normal mode by 100 ms. The control unit 116 may then switch the operating modes based on a user operation. For example, the control unit 116 changes the length of the heating period from 196 ms to 296 ms if the operating mode is switched from normal mode to boost mode in the initial heating period. In this way, the user can switch operating modes from the normal mode to the boost mode at a timing at which a high heating rate is required, such as when it is desired to especially shorten the pre-heating period, or when it is desired to take continuous puffs in a short period of time. According to such a configuration, it is possible to provide a user experience that is more in line with the requirements of the user. Note that various operations are conceivable as the user operation for switching operating modes, such as pressing a button provided on the inhalation device 100, a gesture operation such as shaking the inhalation device 100, operations via an external terminal such as a smartphone, etc.

The notification unit 113 may notify the user of information that is based on the length of the heating period. For example, the notification unit 113 may cause the LED to light up in green in the normal mode and the LED to light up in red in the boost mode. The information that is based on the length of the heating period may be notified by the light emitting pattern defined such as by the light emitting color or the number of LEDs emitting light, as well as by vibrations or sounds.

(4) Other supplementary information

As described in the above variant examples, further control of the length of the heating period may be performed in at least one of the initial heating period and the re-heating period. However, whichever control is performed, it is desirable that the length of the heating period in the re-heating period is shorter than the length of the heating period in the initial heating period.

In the above description, examples were described in which the operation of the heating units 121 is controlled based on the temperature of the heating units 121, but the present disclosure is not limited to such an example. The operation of the heating units 121 may be controlled based on a parameter corresponding to the temperature of the heating units 121. Similarly, the heating profile may comprise target values for parameters corresponding to the temperature of the heating units 121. Parameters corresponding to the temperature of the heating units 121 include the electrical resistance of the heating units 121.

In the above description, an example in which a gradual increase in the temperature of the heating units 121 in the re-heating period was described, but the present disclosure is not limited to such an example. In the re-heating period, the temperature of the heating units 121 may temporarily decrease.

It should be noted that the series of processes performed by each device described in the present description may be realized by using software, hardware, and any combination of software and hardware. Programs constituting the software are prestored on a recording medium (more specifically, a non-transitory computer-readable storage medium) provided internally or externally to each device, for example. When the programs are then executed, for example, by a computer for controlling each device described in the present description, the programs are read into a RAM and executed by means of a processing circuit such as a CPU. The recording medium is, for example, a magnetic disk, an optical disk, a magneto-optical disk, or a flash memory, etc. Furthermore, the computer programs may be distributed via a network, for example, without the use of a recording medium. Furthermore, the computer may be an application-specific integrated circuit such as ASIC, a general-purpose processor which executes functions by reading software programs, or a computer on a server used for cloud computing, etc. Furthermore, the series of processes performed by each device described in the present description may be processed in a distributed manner by multiple computers.

Furthermore, the processing described using flowcharts and sequence diagrams in the present description need not necessarily be implemented in the order depicted. Some processing steps may be implemented in parallel. Furthermore, additional processing steps may be employed and some processing steps may be omitted.

It should be noted that configurations such as the following also fall within the technical scope of the present disclosure.

(1) An aerosol generation system comprising: a heating unit for heating an aerosol source; and

a control unit for controlling the operation of the heating unit based on a parameter corresponding to the temperature of the heating unit, wherein
the control unit controls the operation of the heating unit by repeating, in the stated order, a first step for measuring the parameter of the heating unit and a second step for applying a voltage to the heating unit in a manner determined based on the parameter measured in the first step, and
variably controls the duration of the second step.

(2) The aerosol generation system as set forth in (1), wherein the control unit controls the duration of the second step based on the parameter measured in the first step.

(3) The aerosol generation system as set forth in (1) or (2), wherein the control unit controls the operation of the heating unit based on control information defining a time series transition of a target value of the parameter, and controls the duration of the second step based on the control information.

(4) The aerosol generation system as set forth in (3), wherein a period during which the operation of the heating unit is controlled based on the control information includes, in the stated order:

a first period during which the temperature of the heating unit is increased from or maintained at an initial temperature,
a second period following the first period, during which the temperature of the heating unit decreases, and
a third period following the second period, and
the control unit causes the duration of the second step to differ between the first period and the third period.

(5) The aerosol generation system as set forth in (4), wherein the control unit makes the duration of the second step in the third period shorter than the duration of the second step in the first period.

(6) The aerosol generation system as set forth in (5), wherein the control unit, in the third period, increases the duration of the second step in accordance with the passage of time.

(7) The aerosol generation system as set forth in any one of (4) to (6), wherein the control unit, in the first period, shortens the duration of the second step after the parameter measured in the first step reaches the specific target value.

(8) The aerosol generation system as set forth in any one of (4) to (6), wherein the control unit, in the first period, shortens the duration of the second step in accordance with the passage of time.

(9) The aerosol generation system as set forth in any one of (3) to (8), wherein the control unit controls the duration of the second step based on the difference between the parameter measured in the first step and the target value defined in the control information.

(10) The aerosol generation system as set forth in any one of (1) to (9), wherein the control unit controls the duration of the second step based on a manner of applying voltage to the heating unit in the second step.

(11) The aerosol generation system as set forth in any one of (1) to (10), wherein the control unit controls the duration of the second step based on a user operation.

(12) The aerosol generation system as set forth in any one of (1) to (11), wherein the aerosol generation system further comprises a notification unit for notifying of information that is in accordance with the duration of the second step.

(13) The aerosol generation system as set forth in any one of (1) to (12), wherein the aerosol generation system further comprises a substrate containing the aerosol source.

(14) A control method executed by a computer controlling an aerosol generation system,

the aerosol generation system having
a heating unit for heating an aerosol source,
the control method including:

controlling the operation of the heating unit based on a parameter corresponding to the temperature of the heating unit, wherein
controlling the operation of the heating unit includes
controlling the operation of the heating unit by repeating, in the stated order, a first step for measuring the parameter of the heating unit and a second step for applying a voltage to the heating unit in a manner determined based on the parameter measured in the first step, and
variably controlling the duration of the second step.

(15) A program executed by a computer controlling an aerosol generation system,

the aerosol generation system having
a heating unit for heating an aerosol source,
the program causing the computer to function as
a control unit for controlling the operation of the heating unit based on a parameter corresponding to the temperature of the heating unit, wherein
the control unit controls the operation of the heating unit by repeating, in the stated order, a first step for measuring the parameter of the heating unit and a second step for applying a voltage to the heating unit in a manner determined based on the parameter measured in the first step, and
variably controls the duration of the second step.

[Reference Signs List]

| 100 | Inhalation device |
| 111 | Power source unit |
| 112 | Sensor unit |
| 113 | Notification unit |
| 114 | Memory unit |
| 115 | Communication unit |
| 116 | Control unit |
| 121 | Heating unit |
| 140 | Accommodating portion |
| 141 | Internal space |
| 142 | Opening |
| 143 | Bottom portion |
| 144 | Heat insulating portion |
| 150 | Stick-type substrate |
| 151 | Substrate portion |
| 152 | Mouthpiece portion |

**Claims**

1. An aerosol generation system comprising: a heating unit for heating an aerosol source, and

   a control unit for controlling the operation of the heating unit based on a parameter corresponding to the temperature of the heating unit, wherein
   the control unit controls the operation of the heating unit by repeating, in the stated order, a first step for measuring the parameter of the heating unit and a second step for applying a voltage to the heating unit in a manner determined based on the parameter measured in the first step, and
   variably controls the duration of the second step.

2. The aerosol generation system as claimed in claim 1, wherein the control unit controls the duration of the second step based on the parameter measured in the first step.

3. The aerosol generation system as claimed in claim 1 or 2, wherein the control unit controls the operation of the heating unit based on control information defining a time series transition of a target value of the parameter, and controls the duration of the second step based on the control information.

4. The aerosol generation system as claimed in claim 3, wherein a period during which the operation of the heating unit is controlled based on the control information includes, in the stated order:

   a first period during which the temperature of the heating unit is increased from or maintained at an initial temperature,
   a second period following the first period, during which the temperature of the heating unit decreases, and
   a third period following the second period, and
   the control unit causes the duration of the second step to differ between the first period and the third period.

5. The aerosol generation system as claimed in claim 4, wherein the control unit makes the duration of the second step in the third period shorter than the duration of the second step in the first period.

6. The aerosol generation system as claimed in claim 5, wherein the control unit, in the third period, increases the duration of the second step in accordance with the passage of time.

7. The aerosol generation system as claimed in any one of claims 4 to 6, wherein the control unit, in the first period, shortens the duration of the second step after the parameter measured in the first step reaches the specific target value.

8. The aerosol generation system as claimed in any one of claims 4 to 6, wherein the control unit, in the first period, shortens the duration of the second step in accordance with the passage of time.

9. The aerosol generation system as claimed in any one of claims 3 to 8, wherein the control unit controls the duration of the second step based on the difference between the parameter measured in the first step and the target value defined in the control information.

10. The aerosol generation system as claimed in any one of (1) to (9), wherein the control unit controls the duration of the second step based on a manner of applying voltage to the heating unit in the second step.

11. The aerosol generation system as claimed in any one of claims 1 to 10, wherein the control unit controls the duration of the second step based on a user operation.

12. The aerosol generation system as claimed in any one of claims 1 to 11, wherein the aerosol generation system further comprises a notification unit for notifying of information in accordance with the duration of the second step.

13. The aerosol generation system as claimed in any one of claims 1 to 12, wherein the aerosol generation system further comprises a substrate containing the aerosol source.

14. A control method executed by a computer controlling an aerosol generation system,

the aerosol generation system having
a heating unit for heating an aerosol source,
the control method including:

controlling the operation of the heating unit based on a parameter corresponding to the temperature of the heating unit, wherein
controlling the operation of the heating unit includes
controlling the operation of the heating unit by repeating, in the stated order, a first step for measuring the parameter of the heating unit and a second step for applying a voltage to the heating unit in a manner determined based on the parameter measured in the first step, and
variably controlling the duration of the second step.

15. A program executed by a computer controlling an aerosol generation system,

the aerosol generation system having
a heating unit for heating an aerosol source,
the program causing the computer to function as
a control unit for controlling the operation of the heating unit based on a parameter corresponding to the temperature of the heating unit, wherein
the control unit controls the operation of the heating unit by repeating, in the stated order, a first step for measuring the parameter of the heating unit and a second step for applying a voltage to the heating unit in a manner determined based on the parameter measured in the first step, and
variably controls the duration of the second step.

FIG. 1

FIG. 2

EP 4 602 961 A1

16

FIG. 3

FIG. 4

```
                    ┌──────────────┐
                    │    START     │
                    └──────┬───────┘
                           │
                           ▼
        ┌───────────────────────────────────┐
        │      Receive user operation for   │── S102
        │       instructing start of heating│
        └──────────────┬────────────────────┘
                       │
                       ▼
        ┌───────────────────────────────────┐
        │  Set length of measurement period │
        │  to 4m, set length of heating     │── S104
        │  period to 196 ms, and start      │
        │  heating                          │
        └──────────────┬────────────────────┘
                       │◄──────────────────────┐
                       ▼                   S106 │ NO
              ◇─────────────────◇───────────────┘
              │ Has initial heating │
              │   period ended?     │
              ◇─────────┬───────────◇
                   YES  │
                        ▼
        ┌───────────────────────────────────┐
        │         Stop heating              │── S108
        └──────────────┬────────────────────┘
                       │◄──────────────────────┐
                       ▼                   S110 │ NO
              ◇─────────────────◇───────────────┘
              │ Has intermediate    │
              │ temperature-drop    │
              │   period ended?     │
              ◇─────────┬───────────◇
                   YES  │
                        ▼
        ┌───────────────────────────────────┐
        │  Set length of measurement period │
        │  to 4 ms, set length of heating   │── S112
        │  period to 46 ms, and restart     │
        │  heating                          │
        └──────────────┬────────────────────┘
                       │◄──────────────────────┐
                       ▼                   S114 │ NO
              ◇─────────────────◇───────────────┘
              │ Is end condition    │
              │     satisfied?      │
              ◇─────────┬───────────◇
                   YES  │
                        ▼
        ┌───────────────────────────────────┐
        │        End heating                │── S116
        └──────────────┬────────────────────┘
                       │
                       ▼
                ┌──────────────┐
                │     END      │
                └──────────────┘
```

## INTERNATIONAL SEARCH REPORT

| International application No. |
|---|
| **PCT/JP2022/041233** |

| A. | CLASSIFICATION OF SUBJECT MATTER |
|---|---|

*A24F 40/57*(2020.01)i
FI:  A24F40/57

According to International Patent Classification (IPC) or to both national classification and IPC

| B. | FIELDS SEARCHED |
|---|---|

Minimum documentation searched (classification system followed by classification symbols)

A24F40/57;H05B3/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 2021-503932 A (PHILIP MORRIS PRODUCTS S.A.) 15 February 2021 (2021-02-15) see, in particular, paragraphs [0016]-[0019], [0142], fig. 1-3 | 1-3, 9-10, 13-15 |
| A | entire text, all drawings | 4-8, 11-12 |
| A | JP 2022-520322 A (KT&G CORP.) 30 March 2022 (2022-03-30) entire text, all drawings | 1-15 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 December 2022** | **27 December 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2022/041233**

| Patent document cited in search report | | | Publication date (day/month/year) | Patent family member(s) | | | Publication date (day/month/year) |
|---|---|---|---|---|---|---|---|
| JP | 2021-503932 | A | 15 February 2021 | US | 2020/0367569 | A1 | |
| | | | | see, in particular, paragraphs [0018]-[0021], [0154], fig. 1-3 | | | |
| | | | | WO | 2019/105879 | A1 | |
| | | | | CN | 111356378 | A | |
| | | | | KR | 10-2020-0093588 | A | |
| JP | 2022-520322 | A | 30 March 2022 | WO | 2021/145570 | A1 | |
| | | | | entire text, all drawings | | | |
| | | | | CN | 113412068 | A | |
| | | | | KR | 10-2021-0092591 | A | |

Form PCT/ISA/210 (patent family annex) (January 2015)

**REFERENCES CITED IN THE DESCRIPTION**

**Patent documents cited in the description**

• JP 2022520322 A